# EUROPEAN PATENT APPLICATION

(11) **EP 2 166 359 A1**
(43) Date of publication of application: **24.03.2010**
(21) Application number: 09008325.4
(22) Date of filing: 27.05.2005
(51) Int. Cl.: G01N 33/72

(54) **Assay system with in situ formation of diazo reagent**

(30) Priority: 01.06.2004 US 858673
(62) Divisional of application: 05755084.0
(71) Applicant: Beckman Coulter, Inc., Fullerton, CA 92834-3100 (US)
(72) Inventor: Mu, Xihai, Chino Hills CA 91709 (US); Cernosek, Rose, Mary, Placentia CA 92870 (US)
(74) Representative: Müller-Boré & Partner Patentanwälte

(57) **Abstract**

A kit for detecting bilirubin for a total bilirubin diazo assay is disclosed in which an acidic solution containing a primary aryl amine is combined directly with a nitrite-containing sample solution at approximately neutral pH, resulting in the in situ generation of a diazo compound in the sample solution. The diazo compound reacts with bilirubin in the sample solution to form a colored reaction product that can be measured.

## Description

### CROSS-REFERENCE TO RELATED APPLICATION

The present Application claims the benefit of United States patent application 10/858,673, filed June 1, 2004, entitled "Assay System With In Situ Formation Of Diazo Reagent." The entire disclosure of this application is hereby incorporated herein for all purposes.

### BACKGROUND

The present invention relates to a method of forming a diazo reagent for use in an assay. Diazo reagents are frequently used in clinical applications to detect bilirubin, a breakdown product of hemoglobin. Elevated serum bilirubin levels are observed in a variety of disease conditions, including hemolytic disorders, biliary duct obstruction, cholestasis, hepatitis, and cirrhosis. The quantification of the amount of bilirubin in a patient sample is useful in diagnosing these conditions and in monitoring their treatment.

To detect bilirubin in a sample (such as serum or plasma), the sample can be contacted with a reagent composition which includes a diazonium salt. The diazonium salt reacts with bilirubin to form azobilirubin fragments which can be detected spectrophotometrically. The azobilirubin has an extinction coefficient which is higher than that of bilirubin itself and is thus detectable.

Many diazonium salts have been suggested for use in diazo methods for determining bilirubin. For example, certain 2,4- and 2,5-phenyldiazonium salts and diazotized sulfanilamide have been used for the detection of bilirubin in serum and urine. However, some of these diazonium salts, when dry, are explosively unstable, i.e. subject to shock induced decomposition. The handling of such compounds in connection with performing bilirubin assays can thus be hazardous and these compounds are not used commercially in clinical applications.

Due to the disadvantages of using diazonium salt compounds directly, diazonium (or simply "diazo") reagents for use in detecting bilirubin are generally prepared just prior to use in a detection assay by combining precursor reagents. However, such diazo reagents are unstable and generally must be used within a matter of days or else discarded. A dark yellow color develops in diazo reagent solutions after a short time, and this decay of the diazo reagent can affect the accuracy of a bilirubin assay. In some cases a diazo reagent may be stable for only eight hours, and even solutions with a longer shelf life show the development of a dark yellow color after a few days.

Various solutions have been proposed to improve bilirubin diazo assays. U.S. Patent No. 2,737,501 to Sherman, for example, combines dry powder forms of sodium nitrite and sodium sulfanilate, diazo assay precursors, into a tablet. The tablet is dissolved in solution to then form the diazo reagent just prior to use.

A different approach is discussed in U.S. Patent No. 5,183,762 to Meiattini, in which a sample is first mixed with an acid, after which a nitrite solution is added to the acidified sample solution to form the diazo reagent. The sample solution is buffered at pH 3.1-3.6. However, azobilirubin is not stable at low pH, and proteins in the sample solution may precipitate at such a low pH.

### SUMMARY

The present invention provides an improved method of creating a diazo reagent for use in detecting an analyte in a sample. In this method, a sample solution which includes a nitrite ion and which has a pH of greater than about 5 is first obtained, for example by adding a solution containing a nitrite ion to the sample. This sample solution is next combined with an acidic solution that includes a primary aryl amine, leading to the formation of a diazo reagent in the sample solution. The acidic solution preferably has a pH of less than about 3.5, and in a preferred embodiment has a pH of between about 1 and about 2. The liquid volume of the acidic solution is preferably about 10% or less of the volume of the sample solution. After the formation of the diazo reagent in the sample solution, a spectrophotometric measurement of the sample solution can be obtained.

In another aspect, the present invention provides a method for detecting bilirubin in a sample. This method involves combining a nitrite-containing solution having a pH of greater than about 5 with the sample in order to prepare a sample solution. An acidic solution comprising a primary aryl amine is then added to the sample solution, thereby forming a diazo reagent in the sample solution, after which the presence of azobilirubin can be tested for in the sample solution. The acidic solution preferably has a pH of less than about 3.5, and more preferably between about 1 and about 2. The step of testing for the presence of azobilirubin in the sample can comprise obtaining a spectrophotometric measurement of the sample solution.

In yet another aspect, the present invention comprises a kit for detecting bilirubin in a sample. Such a kit includes a nitrite-containing solution having a pH of greater than about 5; an acidic solution comprising a primary aryl amine; and instructions directing the combination of the nitrite-containing solution with the sample, followed by the addition of the acidic solution to this combination. The acidic solution preferably includes a strong acid, such as phosphoric acid, nitric acid, sulfuric acid or hydrochloric acid, which can be present in the acidic solution at a concentration of between 0.01 M and 0.60 M. The acidic solution also preferably has a pH of less than about 3.5, and more preferably between about 1 and about 2. The primary aryl amine of the acidic solution can be sulfanilic acid, anthranilic acid or another primary aryl amine. The nitrite compound in the nitrite-containing solution can be, for example, sodium nitrite, potassium nitrite, lithium nitrite, calcium nitrite, or magnesium nitrite. The nitrite-containing solution preferably further includes an accelerator such as dyphylline, caffeine, acetate, benzoate, DMSO, TMS, methanol, or gum arabic. The instructions included with the kit preferably direct the testing of blood, plasma, serum or urine with the reagents provided in the kit, for example through the use of an automated analytical instrument.

A further aspect of the present invention is a method of detecting bilirubin in a sample with an automated analytical instrument. In this method the nitrite reagent is automatically delivered into a cuvette of the analytical instrument, and the sample is then automatically added to the nitrite-containing cuvette, thereby forming a sample solution. The instrument then automatically adds an acidic reagent comprising a primary aryl amine to the sample solution, thereby forming a diazo reagent in the sample solution. In a preferred embodiment, a spectrophotometric measurement of the sample solution is next obtained, preferably in an automated manner. The present method allows an automated instrument performing this assay to operate for a period of two weeks or longer without receiving additional nitrite reagent or acidic reagent, due to the stability of the reagents.

### DRAWINGS

These and other features, aspects and advantages of the present invention will become better understood with regard to the following description, appended claims, and accompanying figures where:
Figure 1 is a graph showing the kinetic response of a total bilirubin assay performed according to the present method.
Figure 2 is a graph showing the linearity of the measured instrument response (blank-corrected absorbance) in a total bilirubin assay performed according to the present method.
Figure 3 is a graph comparing the determined bilirubin concentration (mg/dL) of several samples using the present method as compared with a prior art method.

All dimensions specified in this disclosure are by way of example only and are not intended to be limiting. Further, the proportions shown in these Figures are not necessarily to scale. As will be understood by those with skill in the art with reference to this disclosure, the actual dimensions of any device or part of a device disclosed in this disclosure will be determined by their intended use.

### DESCRIPTION OF THE INVENTION

The present invention overcomes drawbacks in prior methods of forming diazo reagents by combining a sample solution that contains a nitrite ion with an acidic primary aryl amine reagent, thereby forming a diazo reagent in situ in the sample solution. A diazo reagent produced in this way contacts any bilirubin present in the sample within a few minutes of its formation, thus obviating issues relating to the short shelf life of diazo reagents. The sample solution is also maintained at an essentially non-acidic pH during the present method, thus avoiding unwanted protein precipitation. The inventors have found moreover that bilirubin assays performed according to the present method show good linearity, precision, accuracy, and a fast kinetic response.

As used herein, the term "diazo reagent" means an aromatic diazonium salt formed from the reaction of a primary aryl amine and a nitrite compound. The term "nitrite compound" denotes a compound comprising an -NO₂ group (such as a salt or ester of nitrous acid), and "nitrite ion" means NO₂⁻. A "nitrite-containing solution" is an aqueous solution containing a nitrite ion. "Nitrite reagent" means a nitrite-containing solution for creating a diazo reagent. "Sample solution" denotes a sample admixed with a nitrite-containing solution.

The term "primary aryl amine" as used herein means a compound comprising an amino group (a derivative of ammonia) in which only one atom of hydrogen has been replaced by an aromatic compound including one or more benzene rings. The primary aryl amines must be capable of reacting with a nitrite to form a diazo compound. The term "acidic primary aryl amine reagent" is used to denote a reagent for producing a diazo reagent which comprises a primary aryl amine at a pH of about 5 or less.

As used herein, the term "comprise" and variations of the term, such as "comprising" and "comprises," are not intended to exclude other additives, components, integers or steps.

### Nitrite Reagent

The present invention preferably involves the use of only two reagents, a nitrite reagent and an acidic primary aryl amine reagent. The nitrite reagent comprises a nitrite ion in solution. The nitrite ion is generally derived from a compound having the formula Y(NO₂)ₘ, where Y is preferably an alkali earth or alkaline metal such as sodium (Na⁺), potassium (K⁺), lithium (Li⁺), calcium (Ca⁺⁺), or magnesium (Mg⁺⁺) and m is the absolute value of the valence of Y. Preferably, the nitrite ion is present in aqueous solution at a concentration of between about 7 mM and 75 mM, for example about 55 mM. The use of higher concentrations is also possible and will not adversely affect the formation of a diazo reagent. A preferred nitrite compound is sodium nitrite (NaNO₂), which can be present in solution at a concentration of about 0.05 - 0.5% w/v sodium nitrite.

The nitrite reagent has a pH greater than about 5, preferably greater than about 6, more preferably between about 7 and about 8. While the formation of a diazo reagent according to the present invention can occur when the nitrite reagent and/or the sample solution have a pH higher than 8, the use of higher pH solutions is not preferred. Since the formation of the diazo reagent is believed to occur in a locally and/or transiently more acidic environment within the nitrite reagent and/or the sample solution when the acidic primary aryl amine reagent is added, the use of nitrite reagents and sample solutions at higher pH would require the use of higher volumes of the acidic reagent or an acidic reagent at lower pH than is necessary when the nitrite reagent and/or the sample solution have a pH of between about 7 and 8, for example.

Buffers can be included in the nitrite reagent to establish and/or maintain a desired pH. For example, sodium acetate having a concentration of between about 5 and 100 g/L can be used. Higher concentrations will not adversely affect the reaction forming the diazo reagent, but preferably about 50 g/L sodium acetate is included. When sodium acetate is used, it serves the dual role of buffering the nitrite reagent and accelerating the reaction of the diazo reagent with bilirubin as described below. Other buffers, such as phosphate buffers, can also be used in concentrations sufficient to establish the proper pH. Buffering compounds used in the nitrite reagent also serve to buffer the sample solution.

### Acidic Reagent

The acidic primary aryl amine reagent can include any of a number of primary aryl amines, such as 2,4-dichloroaniline, p-aminobenzene sulfonic acid, anthranilic acid, p-chloroaniline, 2-methoxy-5-nitroaniline, and p-toluidine. Aminobenzene derivatives such as anthranilic acid and p-aminobenzene sulfonic acid (also called sulfanilic acid) are preferred, with sulfanilic acid being particularly preferred.

Primary aryl amines are included in the acidic reagent in sufficient quantities such that the amount of diazo reagent formed therefrom is adequate to cause substantially complete conversion of the bilirubin expected to be present in a sample solution to azobilirubin. It is therefore recommended that the acidic primary aryl amine reagent have a concentration of primary aryl amine of about 5 to 50 mM, in particular in assays involving plasma, serum or blood. In a preferred system, the acidic primary aryl amine reagent contains between 25 and 30 mM, or about 0.5% (w/v) sulfanilic acid.

The acidic primary aryl amine reagent is preferably maintained at a pH of less than about 4, more preferably less than about 3.5, and even more preferably between about 1 and 2. The use of an acidic primary aryl amine reagent at lower pH allows a smaller volume of the acidic primary aryl amine reagent to be used to form the diazo reagent. A strong acid, i.e. one which completely or substantially completely dissociates in water, such as hydrochloric acid is therefore preferably included in an acidic primary aryl amine reagent in order to provide a desired acidic pH. Suitable acids for use in the acidic primary aryl amine reagent include phosphoric acid, nitric acid, sulfuric acid and hydrochloric acid, though others are also possible. A preferred acid is hydrochloric acid, which can be used in a concentration of from about 0.01 to 0.60 M (or higher), preferably about 0.1 M.

### Reaction Accelerators

The nitrite reagent and/or the acidic primary aryl amine reagent also preferably include what are known as a "diazo bilirubin promoters," "accelerating agents" or simply "accelerators". In the absence of such accelerators, the reaction between a diazo reagent and bilirubin, and hence the development of a detectable colored compound, is slower. Accelerators useful in the present invention include dyphylline, caffeine, acetate (e.g., sodium acetate), benzoate (e.g., sodium benzoate), and gum arabic. Salts, esters, and other derivatives of these compounds can also be used. Such compounds are used in concentrations known to the art. Such accelerators are generally present in the nitrite reagent rather than in the acidic reagent.

At low pH, dimethylsulfoxide (DMSO) can also be used as an accelerator, as disclosed by Walters and Gerarde in Microchem. J., 15:231 (1970). However, DMSO is somewhat viscous and therefore may not be readily adaptable for use in an automated system. Methanol can also be used, but is less favored when the sample to be tested comprises plasma or serum because it can cause precipitation of serum proteins. In addition, tetramethylene sulfone (TMS) can also be used as an accelerator, and may prevent the precipitation of serum proteins in a total bilirubin assay when methanol is also present.

Several accelerators can be used together in the reagents of the present invention. For example, the collection of accelerators can be of the type proposed by Jendrassik and Grof in Biochem. Z., 297:81 (1938) and comprise 0.39M sodium benzoate, 0.19M caffeine, and 0.69M sodium acetate.

### Other Reagents

Salts such as sodium chloride can also be included in the nitrite reagent and/or the acidic primary aryl amine reagent. When salts are present in a concentration of 1 M to saturation, optimally about 2 M, they help to stabilize the diazo reagent. However, since the diazo reagent in the present invention is created in a solution containing a sample to be assayed, in most embodiments of the invention it is not important to provide means for stabilizing the diazo reagent.

Other components can also be included in the reagents used in the present invention. A preservative such as EDTA or other preservatives known to the art can be used, for example, in the nitrite reagent.

To be stable, the nitrite and acidic primary aryl amine reagents should be able to create a sufficient quantity of diazo reagent when used to perform a total bilirubin diazo assay after storage at 15-30°C (i.e., room temperature) for 2 years. Reagents stored on-instrument (i.e., in a chamber or container associated with an analytical instrument) may typically be exposed to temperatures of between 2 and 40°C, and commonly remain on an instrument in commercial use for about two months. Stability of the reagents on-instrument is important commercially, so a stable reagent should therefore retain stability when stored at up to 40°C for about two months. Reagents are generally not used after being on-instrument for more than two months, in order to minimize the possibility of evaporation or contamination of the reagents.

### Detecting Bilirubin

To detect the presence of bilirubin in a sample according to the present invention, an aliquot of the nitrite reagent is first combined with a biological sample to form a sample solution. The nitrite reagent can be added to a container or reaction vessel manually, such as with a pipetter, or can be injected or otherwise added automatically by an analytical instrument, followed by the addition of the sample. Alternatively, the sample can be added to the nitrite reagent. The sample solution should be at a pH greater than about 5. If the pH of the sample is lower than this prior to the addition of the nitrite reagent, the nitrite reagent should contain a sufficient amount of a buffering compound to raise the pH of the sample solution.

The sample can comprise, for example, any material suspected of possibly containing bilirubin. Most frequently the sample comprises blood, plasma, serum, or urine. Samples can be dry (such as a lyophilized sample), in which case it should be solubilized or suspended either in the nitrite reagent or in another aqueous solution prior to combination with the nitrite reagent.

Once the sample solution has been formed, the acidic primary aryl amine reagent is added to this solution to form a diazo reaction mixture. The primary aryl amine in this mixture reacts with a nitrite ion to form the diazo reagent. A molar excess of the diazo reagent as compared with the amount of bilirubin present (or suspected of being present) should be produced in order to detect all possible bilirubin in the sample. Since one mole of the primary aryl amine reacts with one mole of nitrite ion to form the diazo reagent, there must be a sufficient molar quantity of each chemical species to produce enough diazo reagent to react with an expected amount of bilirubin in a sample. Preferably at least 0.5 mM of diazo reagent is produced in order to cover the range of bilirubin concentrations encountered in clinical usage. A molar excess of either the primary aryl amine or the nitrite ion is possible. In one embodiment, the acidic reagent contains one mole of a primary aryl amine for approximately every two moles of nitrite ion present in the sample solution.

The diazo-forming reaction preferably occurs at normal operating temperatures for conducting clinical analyses, generally between 2 and 40°C, but other temperatures are possible. Upon the initial addition of the acidic primary aryl amine reagent to the sample solution, the pH of this diazo reaction mixture is believed to be locally (that is, in pockets within the sample solution) and/or transiently at a sufficiently low pH to allow the diazo reagent to be formed. However, after formation of the diazo reagent, the diazo-containing sample solution is at a pH of greater than about 5, generally not below pH 6.

The diazo reagent reacts with any bilirubin present in the sample solution almost immediately after the diazo compound is formed, and the complete reaction of all bilirubin in a sample generally takes place in less than 5 minutes. The final color of the azobilirubin solution produced by the reaction is stable for at least one hour at room temperature, particularly if direct sunlight is avoided.

The diazotized sample solution is then tested for the presence of azobilirubin using a spectrophotometer. Using light with a wavelength of around 520 nm to measure azobilirubin in the sample solution is preferred and generally results in greater assay sensitivity than the use of other wavelengths, though other wavelengths (such as 600 nm) can also be used. If the sample is not already in a container comprising at least one transparent wall or surface, it should be transferred into such a container to facilitate spectrophotometric detection of the azobilirubin. Instruments for performing a spectrophotometric analysis of diazotized bilirubin include the SYNCHRON CX and SYNCHRON LX clinical analyzers (made by Beckman Coulter, Inc., 4300 N. Harbor Boulevard, Fullerton, CA 92834), though other spectrophotometers or devices which include a spectrophotometer can be used. The light absorbance measured by a spectrophotometer is converted into bilirubin concentration information by known methods.

Spectrophotometer measurements are known to sometimes be less accurate when an analyte is present in a sample at low concentration. The volumetric amount of nitrite reagent and acidic primary aryl amine reagent added to a sample which is already in solution is therefore preferably minimized so as not to significantly dilute the solution and lower the concentration of bilirubin to a level at which spectrophotometer measurements become less accurate. In one embodiment, the volumetric amount of acidic primary aryl amine reagent added to a sample solution is about 10% or less of the liquid volume of the sample solution, and more preferably 5% or less. For example, 200 µl of a sample solution in an analytical instrument can preferably have added to it about 10 µl of an acidic primary aryl amine reagent.

### Use of Analytical Instruments

The present assay can advantageously be performed with an automated analytical instrument with less user input compared to prior assays. The term "automated" and "automatically" are used herein to refer to actions performed directly by an instrument rather than by a human operator, though an operator normally at least provides instructions to the instrument or performs an action (such as placing a sample in the instrument) which triggers the instrument to perform such automated actions in connection with performing the assay. In an especially preferred embodiment, not only is the formation of the diazo reagent automated, but the spectral analysis of the sample is automated as well. For example, the sample, nitrite reagent, and acidic primary aryl amine reagent can all be mixed in the cuvette of a clinical analyzer and then subjected to spectral analysis without further input or attention from an operator of the instrument.

The present invention also facilitates the performance of bilirubin measurement using a clinical analyzer because the necessary reagents can have an on-instrument shelf life of at least two months. For example, the reagents described in Example 1 below are stable for more than two months on-instrument, and for more than two years on the shelf (in a sealed container, at room temperature).

To perform an assay according to the present invention with an automated instrument, the nitrite and acidic primary aryl amine reagents can be placed into two compartments of a reagent cartridge, after which the cartridge is loaded onto the instrument. The samples being assayed are loaded into a cup or container in the instrument, which can be on a rack. Probes from the analyzer then automatically obtain and deliver the nitrite reagent and the sample to a measurement cuvette, after which an appropriate amount of the acidic primary aryl amine reagent is also automatically added. Preferably, the cuvette holding the diazotized sample is then automatically subjected to spectrophotometric analysis. With this system, the foregoing assay steps (with or without automated spectrophotometry) can be performed at any point over a longer period of time than the 1 to 3 day life of a typical diazo reagent, without needing to throw away or change out the nitrite or acidic primary aryl amine reagents due to the stability of these reagents. Such assays can thus be performed over a period of 4 days, 1 week, 2 weeks or even up to two months (or longer, depending on the storage conditions of the reagents) without adding new reagents to the instrument, depending only on how quickly the assay reagents are used up.

A number of commercially available clinical analyzers can be used to perform the present assay. For example, the SYNCHRON CX and SYNCHRON LX clinical analyzers made by Beckman Coulter can be configured to mix a sample, the nitrite reagent, and the acidic reagent used in the present method and also perform a spectral analysis.

### Kits

The nitrite reagent and/or the acidic primary aryl amine reagent can be sold as a kit for use in determining the presence of bilirubin in a sample. Such a kit includes the nitrite reagent and the acidic primary aryl amine reagent as described above, and preferably also includes instructions for using these reagents. The instructions would direct the combining of the nitrite reagent with a sample to be tested, such as with a pipetter or an automated analytical instrument, followed by the addition of the acidic reagent to the sample solution. Such instructions then preferably direct that a spectrophotometer be used to measure the diazotized sample solution and that absorbance data be recorded, either by an individual performing the assay or by an automated analytical device.

In a kit for use with an automated analytical instrument, such as a clinical analyzer, the instructions preferably direct the following steps for performing an assay according to the present invention:
(1) placing a sample (such as blood, plasma, serum and/or urine) in a container adapted to be used with the automated instrument or into a compartment of the instrument;
(2) operating the instrument so as to direct it to sequentially mix the nitrite reagent with the sample and then with the acidic primary aryl amine reagent in a container or compartment of the instrument; and
(3) operating the instrument so as to direct it to perform a spectrophotometric measurement of the sample solution after the addition of the nitrite reagent and the acidic primary aryl amine reagent.

Reagents can be added directly to the sample-containing container adapted to be used with the automated instrument or the compartment of step (1), or alternatively a probe can obtain a predetermined amount of a sample and dispense it into a further container or compartment of the instrument where the diazo reagent will be formed. Steps (2) and (3) can also be performed (and directed in the kit instructions to be performed) through a single instruction (such as the push of a button or other mechanism). An instrument can also optionally be configured to automatically perform steps (2) and (3) upon receiving a container containing a sample or upon the addition of a sample into a chamber of the instrument, in which case the kit instructions so direct. After the foregoing steps the kit instructions preferably direct the obtaining of absorbance data with a spectrophotometer, converting the absorbance data to bilirubin concentration data, and recording the bilirubin concentration of a sample (either automatically or manually by an instrument operator).

### Example 1: Reagents

Table 1 below discloses a nitrite reagent and an acidic primary aryl amine reagent according to the present invention.

**Table 1. Composition of Total Bilirubin Assay Reagents**

| Nitrite Reagent | | |
|---|---|---|
| **Material** | | **Concentration, g/L** |
| Sodium Acetate | | 53.57 |
| Sodium Benzoate | | 0.059 |
| Sodium Nitrite | | 3.853 |
| Caffeine | | 1.00 |
| EDTA | | 0.564 |
| pH | | 7.5 |

| Acidic Reagent | | |
|---|---|---|
| **Material** | **Concentration, g/L** | |
| Hydrochloric Acid | (0.1 M) | |
| Sulfanilic Acid | 4.81 | |
| Sodium Chloride | 107.3 | |
| pH | <1.0 | |

### Example 2: Reagent Stability

The reagent solutions described in Example 1 were stored at two different temperatures for 7 days and then used to test for the total bilirubin absorbance of the samples. One portion of the reagent solutions was stored at 2-8°C as control, while the other was stored at 45°C. The 45°C data for stability after one week is predictive of stability for two years at 2-8°C.

As shown in Table 2 below, the reagent solutions stored at 45°C result in virtually the same absorbance measurements as the control reagents stored at 2-8°C, indicating that these reagents are stable. The measurements shown are absorbance data collected on a SYNCHRON LX Clinical Chemistry System made by Beckman Coulter, Inc.

**Table 2. Reagent Stability**

| Sample | Absorbance, in mA 2-8°C (control) | Absorbance, in mA 45°C (test) |
|---|---|---|
| Sample 1 | 20.8 | 20.5 |
| Sample 2 | 115.8 | 115.9 |
| Sample 3 | 217.2 | 215.9 |

| | | |
|---|---|---|
| (1 mA = milli-absorbance unit) | | |

### Example 3: Kinetic Response

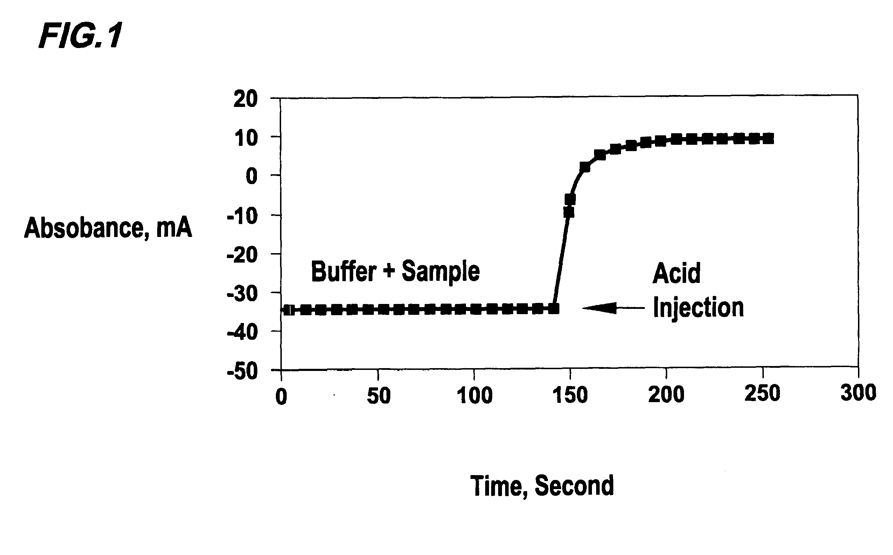

The kinetic response of a total bilirubin assay according to the present method is shown in Figure 1. Ten microliters of a sample from a patient were combined with 200 µl of the nitrite reagent shown in Table 1 in a cuvette in a SYNCHRON LX Clinical Chemistry System. Ten microliters of the acidic reagent shown in Table 1 were then injected into the cuvette. The instrument obtained a spectrophotometer reading at 520 nm, 72 seconds after injection of the acidic reagent. Figure 1 shows that the light absorbance by the spectrophotometer reaches a steady-state within about 30 seconds after the injection of the acidic primary aryl amine reagent.

### Example 4: Assay Linearity

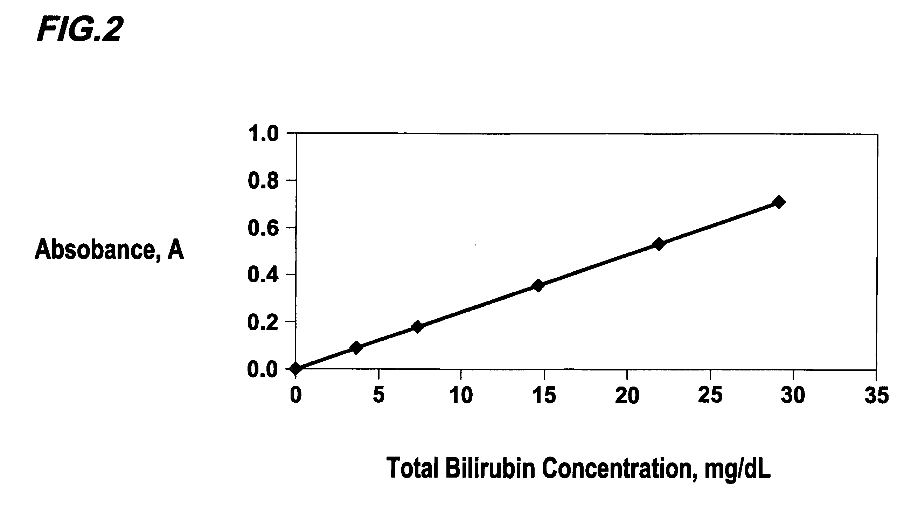

The linearity of the present bilirubin assay, i.e. how linear the determined bilirubin concentration versus the measured absorbance is at different dilutions, was tested under the same experimental conditions as described in Example 3. Bilirubin concentrations of up to 30 mg/dL were measured using a SYNCHRON LX Clinical Chemistry System and the results were plotted in Figure 2. The equation of the line shown in Figure 2 (y=0.0247x - 0.0001) has an R² of 0.9999, which shows good linearity in the present method. The concentration range measured in this experiment is the typical range found in clinical samples.

### Example 5: Assay Precision

Spectrophotometer measurement precision (i.e., how large the coefficient of variance, %CV, of the measurements is) varies the most at low analyte concentrations. Therefore, measurements were taken of a sample having a relatively low bilirubin concentration (0.62 mg/dL) to measure the within-run imprecision in instrument measurements using the method of the present invention. The results, shown in Table 3 below, demonstrate good precision using the present method.

**Table 3. Total Bilirubin Within-Run Imprecision On Beckman SYNCHRON LX**

| | | |
|---|---|---|
| Run #1 | Mean (mg/dL) | 0.62 |
| | SD* (mg/dL) | 0.014 |
| | CV(%) | 2.26 |
| Run #2 | Mean (mg/dL) | 0.62 |
| | SD (mg/dL) | 0.013 |
| | CV(%) | 2.04 |
| Run #3 | Mean (mg/dL) | 0.62 |
| | SD (mg/dL) | 0.013 |
| | CV(%) | 2.09 |

| | | |
|---|---|---|
| * SD = Standard Deviation | | |

### Example 6: Comparative Testing

A set of bilirubin-containing samples was tested both according to the present method and according to a prior method of analysis. In the prior method, 3 reagents are required: (A) a physiological buffer, (B) a sulfanilic acid-containing reagent (containing 0.1 M HCl , 4.81 g/L sulfanilic acid, and 107.31 g/L sodium chloride), and (C) a nitrite ion-containing reagent (comprising 72.35 g/L sodium nitrite and 0.896 g/L EDTA sodium salt). Reagents B and C (10.8 mL of reagent B + 0.1 mL of reagent C) were mixed by the instrument operator to form the diazo reagent just before loading the diazo reagent onto the analytical instrument, due to the instability of this diazo reagent. Following this, 25 µl of the diazo reagent (the mixture of reagents B and C) was combined with 255 µl of a physiological buffer by the SYNCHRON LX system to form a reaction solution, and 8 µl of the sample was added to the reaction cuvette. Absorbance data at 520 nm was obtained 128 seconds after sample injection.

The same samples were then tested according to the present method. In this case, 10 ul of each of the same samples was added to 200 µL of the nitrite-containing solution of Example 1 by the SYNCHRON LX system to form sample solution. Then, 10 µl of the acidic reagent of Example 1 was added to the reaction cuvette, and absorbance data at 520 nm were obtained 72 seconds after acid reagent injection.

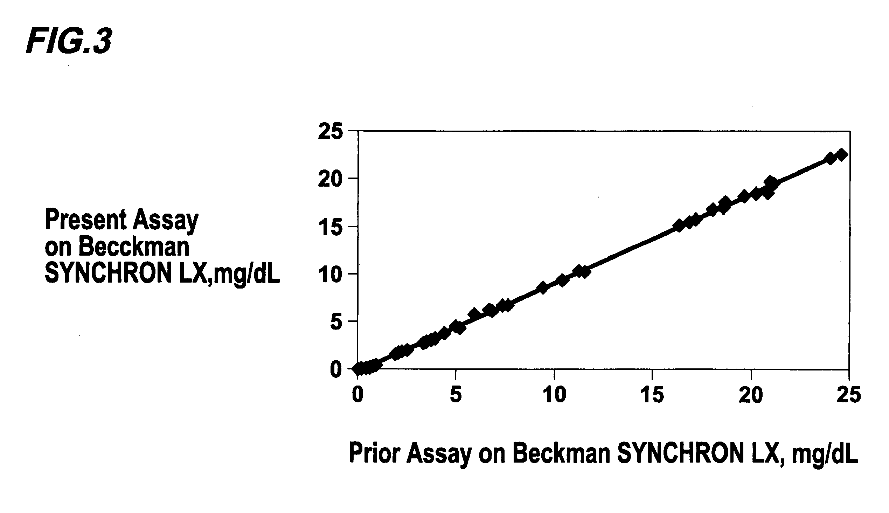

The absorbance data for the samples assayed by both methods were recorded, and the corresponding bilirubin concentration data were plotted in Figure 3. The equation of the line shown in Figure 3 (y=0.9515x - 0.2858) has an R² of 0.9994 and thus shows good linearity. The slope and intercept also show good agreement for the results obtained using both the present method and the prior bilirubin detection method.

Although the present invention has been discussed in considerable detail with reference to certain preferred embodiments, other embodiments are possible. Therefore, the scope of the appended claims should not be limited to the description of preferred embodiments contained in this disclosure. All references cited herein are incorporated by reference to their entirety.

## Claims

1. A kit for detecting bilirubin in a sample, comprising:
(a) a nitrite-containing solution having a pH of greater than 5;
(b) an acidic solution comprising a primary aryl amine; and optionally,
(c) instructions for using reagents (a) and (b), wherein the instructions direct the combining of the nitrite-containing solution with a sample to be tested, followed by the addition of the acidic solution to the combination of the nitrite-containing solution and the sample.

2. The kit of claim 1, wherein the acidic solution comprising a primary aryl amine includes a strong acid, preferably a strong acid comprising phosphoric acid, nitric acid, sulfuric acid, or hydrochloric acid.

3. The kit of claim 2, wherein the strong acid is present in the acidic solution comprising a primary aryl amine at a concentration of between 0.01 M and 0.60 M.

4. The kit claim 1, wherein the acidic solution comprising a primary aryl amine has a pH of less than 4, preferably a pH of between 1 and 2.

5. The kit of any one of claims 1 to 4, wherein the primary aryl amine comprises sulfanilic acid, anthranilic acid, 2,4-dichloroaniline, p-chloroaniline, 2-methoxy-5-nitroaniline, or p-toluidine.

6. The kit of any one of claims 1 to 5, wherein the acidic solution comprises the primary aryl amine at a concentration of between 5 mM and 50 mM.

7. The kit of any one of claims 1 to 6, wherein the nitrite-containing solution includes a nitrite compound comprising sodium nitrite, potassium nitrite, lithium nitrite, calcium nitrite, or magnesium nitrite.

8. The kit of claim 7, wherein nitrite ion in the nitrite-containing solution is present at a concentration of between 7 mM and 75 mM.

9. The kit of any one of claims 1 to 8, wherein the nitrite-containing solution, the acidic solution comprising a primary aryl amine, or both, include an accelerator.

10. The kit of claim 9, wherein the accelerator comprises dyphylline or a derivative thereof, caffeine or a derivative thereof, acetate or a derivative thereof, benzoate or a derivative thereof, gum arabic or a derivative thereof, dimethylsulfoxide (DMSO), tetramethylene sulfone (TMS), methanol, or a combination thereof.

11. The kit of any one of claims 1 to 10, wherein the nitrite-containing solution comprises a buffer, preferably a buffer comprising a sodium acetate buffer or a phosphate buffer.

12. The kit of any one of claims 1 to 11, wherein the instructions direct the testing of blood, plasma, serum, or urine with the solutions provided in the kit.

13. The kit of claim 12, wherein the instructions further direct the use of an automated analytical instrument.

14. The kit of claim 13, wherein the instructions direct steps that include:
(a) placing a sample in a container adapted to be used with an automated instrument or into a compartment of the instrument;
(b) operating the instrument so as to direct it to sequentially mix the nitrite-containing solution with the sample and then with the acidic solution comprising a primary aryl amine in a container or compartment of the instrument; and
(c) operating the instrument so as to direct it to perform a spectrophotometric measurement of the sample solution after the addition of the nitrite-containing solution and the acidic solution comprising a primary aryl amine.

15. The kit of any one of claims 1 to 14, wherein the nitrite-containing solution and the acidic solution comprising a primary aryl amine are stable after storage at 15-30°C (room temperature) for at least 2 years, or after storage on an analytical instrument at temperatures up to 40°C for at least 2 months.
